# FASCICULE DE BREVET EUROPEEN

(11) **EP 0 922 221 B1**
(45) Date de publication et mention de la délivrance du brevet: **19.03.2003**
(21) Numéro de dépôt: 97938957.4
(22) Date de dépôt: 29.08.1997
(51) Int. Cl.: G01N 33/04

(54) **METHODE POUR EVALUER LE TRAITEMENT THERMIQUE AUQUEL EST SOUMIS UN ALIMENT PROTEIQUE TEL QU'UN LAIT**
VERFAHREN ZUR AUSWERTUNG DER THERMISCHEN BEHANDLUNG EINES EIWEISSHALTIGEN LEBENSMITTELS, WIE ETWA MILCH
METHOD FOR EVALUATING THE HOT TREATMENT TO WHICH A PROTEINIC NUTRIENT SUCH AS MILK IS SUBJECTED

(30) Priorité: 30.08.1996 FR 9610603
(43) Date de publication de la demande: 16.06.1999
(73) Titulaire: INSTITUT NATIONAL AGRONOMIQUE PARIS-GRIGNON ( INA-PG), 75005 Paris (FR)
(72) Inventeur: BIRLOUEZ-ARAGON, Inès, F-95120 Ermont (FR)
(74) Mandataire: Hammond, William
(86) Numéro de dépôt international: FR9701533
(87) Numéro de publication internationale: WO98009165

(56) Documents cités:
- EP-A- 0 343 064
- EP-A- 0 422 981
- EP-A- 0 573 054
- DD-A- 249 974
- DATABASE WPI Section Ch, Week 8905 Derwent Publications Ltd., London, GB; Class D12, AN 89-038008 XP002030930 & SU 1 411 666 A (MEAT IND RES INST) , 23 juillet 1988
- SOVIET INVENTIONS ILLUSTRATED Section Ch, Week 44 15 décembre 1982 Derwent Publications Ltd., London, GB; Class B04, AN 94698 XP002030931 & SU 894 496 A (EREV MEDICAL INST) , 30 décembre 1981

## Description

La présente invention est relative à une nouvelle méthode pour évaluer le traitement thermique auquel est soumis un aliment protéique tel qu'un lait.

Actuellement, l'homme du métier dispose de quelques méthodes d'évaluation du traitement thermique d'un lait dont deux sont officielles : la mesure du lactulose dans les laits soumis à un procédé U.H.T., d'une part, et la mesure de la bêta-lactoglobuline pour les laits pasteurisés, d'autre part. Ces deux techniques de dosage utilisent la chromatographie liquide haute performance (H.P.L.C.). La première de ces méthodes permet de maîtriser le traitement U.H.T. mais avec un décalage d'au moins trois heures, ce qui interdit toute intervention rapide sur les paramètres du traitement U.H.T. La seconde permet d'apprécier la qualité d'un lait pasteurisé, mais également avec un décalage trop important dans le temps.

La mise en oeuvre de ces deux procédés ne permet pas à l'industriel d'intervenir rapidement sur le procédé en cours : un certain litrage de lait aura donc subi un traitement thermique (U.H.T., pasteurisation) dans de mauvaises conditions, avant qu'une correction du procédé ne puisse être faite.

L'homme du métier connaît également d'autres méthodes notamment pour mesurer la dégradation des protéines telles que celle présentes dans des laits chauffés. En particulier, on dose des produits issus de la réaction de Maillard tels que la furosine.

Ces méthodes de l'art antérieur, dont la fiabilité ne saurait être remise en cause, présentent notamment trois inconvénients majeurs : comme déjà explicité ci-dessus, elles sont lentes et les résultats ne sont connus qu'après au moins un délai de trois heures ; elles ne permettent de doser qu'une vingtaine d'échartillons par jour au maximum, ce qui n'est guère suffisant pour suivre un procédé continu ; et elles sont relativement coûteuses.

Aussi un des buts de la présente invention est-il de fournir une méthode pour évaluer le traitement thermique auquel est soumis un aliment protéique tel qu'un lait, qui permet d'obtenir des résultats dans des délais brefs compatibles avec les exigences des industriels.

Un autre but de la présente invention est de fournir une telle méthode qui permet le dosage d'environ une centaine d'échantillons par jour.

Un but supplémentaire de l'invention est de fournir une méthode de ce genre qui permet d'évaluer l'effet d'un traitement thermique sur la qualité nutritionnelle des protéines d'un aliment tel que celles du lait.

Ces buts, ainsi que d'autres qui apparaîtront par la suite, sont atteints par une méthode pour évaluer le traitement thermique auquel est soumis un aliment protéique tel qu'un lait, qui est caractérisée, selon la présente invention, par le fait qu'elle comprend les étapes suivantes :
a) on prélève un échantillon de l'aliment auquel on ajoute un tampon de force ionique et de pH appropriés pour obtenir, d'une part, un précipité de protéines dénaturées par le traitement thermique et, d'autre part, un surnageant transparent qui renferme des protéines encore solubles ;
b) on dose le tryptophane présent dans le surnageant ; et
c) on dose les sous-produits fluorescents issus de la réaction avancée de Maillard présents dans le surnageant.

De préférence, le tryptophane présent dans le surnageant est dosé par sa fluorescence ; par exemple, on effectue la mesure à une longueur d'onde d'excitation de 290 nm et à une longueur d'onde d'émission comprise entre 330 et 350 nm pour obtenir un maximum de sensibilité. Le dosage du tryptophane pourrait être également effectué par son absorption U.V.

Avantageusement, le dosage des sous-produits, peptidiques, fluorescents issus de la réaction avancée de Maillard présents dans le surnageant, est réalisé avec une longueur d'onde d'excitation de 350 nm et d'émission comprise entre 420 et 440 nm pour obtenir un maximum de sensibilité

Afin de mieux mettre en évidence les conditions de mise en oeuvre de la méthode selon la présente invention ainsi que ses avantages, l'homme du métier se reportera aux exemples ci-après.

Il sera auparavant rappelé qu'en fonction du traitement thermique les protéines présentes dans un aliment protéique tel qu'un lait, subissent des transformations qui permettent de contrôler ce procédé thermique.

Selon la présente invention on a choisi, d'une part, le tryptophane comme marqueur du taux de dénaturation des protéines et, d'autre part, les produits fluorescents issus de la réaction avancée de Maillard, qui altère la qualité nutritionnelle de la protéine, comme reflet de l'intensité de la réaction entre la lysine et les produits à groupement(s) carbonyle(s) tels que les sucres et les lipides peroxydés.

### Exempte 1 : Lait

On prépare un échantillon de mesure comme suit. 500 µl de lait ayant subi un traitement thermique sont mélangés à 4500 µl de tampon acétate de sodium 100 mM à pH 4,6. Après agitation au vortex, le surnageant transparent est obtenu par centrifugation à 9000 tours par minutes pendant 10 minutes.

Dans une cuve de fluorescence en acryle, 250 µl du surnageant sont dilués avec 2250 µl d'eau. La fluorescence est mesurée sur un spectrofluorimètre commercialisé sous la dénomination Spex par la société Jobin-Yvon ou tout autre fluorimètre équivalent, aux longueurs d'onde ci-après :
- longueur d'onde d'excitation : 290 nm
- longueur d'onde d'émission: 340 nm.

Les résultats en fonction de divers traitements thermiques sont regroupés dans le tableau I ci-après avec ceux obtenus par les méthodes traditionnelles.

**TABLEAU I**

| Méthode de mesure | Invention | | Furosine (mg/100g) | βlactoglobuline (mg/L) | Lactulose (mg/L) |
|---|---|---|---|---|---|
| | Trp UR | 100* (PM/Trp) | | | |
| **Pasteurisés** | | | | | |
| moyenne (n=9) | **2,87** | **14,43** | **9,50** | **2079** | 0 |
| **écart-type** | **0,90** | **5,78** | **0,93** | **1210** | |
| min/mxm | 2,5/4,2 | 10,4/18,7 | 7,1/10,4 | 1401/3429 | |

| **UHT directs** | | | | | |
|---|---|---|---|---|---|
| moyenne (n=9) | 0,679 | **22,15** | **55,02** | **758** | **138,9** |
| écart-type | 0,137 | **7,37** | **24,22** | **538** | **91,7** |
| min/mxm | 0,51/0,89 | 16,7/26,6 | 41,5/58,0 | 240/1204 | 72/171 |

| **UHT Indirects** | | | | | |
|---|---|---|---|---|---|
| moyenne (n=33) | **0,499** | **40,33** | **160,5** | **89,3** | **568,2** |
| écart-type | **0,160** | **11,71** | **54,1** | **35,4** | **325,3** |
| min-mxm | 0,17/0,93 | 31,6/45,7 | 132,9/176,2 | 43,4/112,7 | 365,3/597,5 |
| Trp : tryptophane | | | | | |
| 100* PM/Trp : Rapport de la fluorescence des produits de Maillard à celle du tryptophane | | | | | |

La fluorescence du tryptophane (Trp) est fortement corrélée (r=0,98) à la concentration de la β-lactoglobuline, méthode de référence pour caractériser les laits pasteurisés. Elle permet ainsi de différencier très rapidement un lait pasteurisé d'un lait UHT.

Le rapport entre la fluorescence des produits de Maillard (PM) sur la fluorescence du tryptophane (100*PM/Trp) constitue un index thermique global qui fait également intervenir cette autre réaction essentiellement présente dans les laits UHT, surtout de type indirect. Celle-ci est bien corrélée à la furosine (r=0,82) et est inversement proportionnelle à la qualité nutritionnelle (teneur en l'acide-aminé essentiel lysine) des protéines de lait chauffées.

L'ensemble de ces deux mesures renseigne rapidement sur le type de traitement thermique subi par le lait et la qualité de ce traitement.

Du fait de la rapidité de ce procédé, on peut, d'une part, mesurer une centaine d'échantillons par jour et, d'autre part, intervenir rapidement afin de permettre la production d'un lait aux valeurs nutritives maximales, et ce à moindre coût de fonctionnement.

### Exemple 2 : Soja

La présente méthode peut aussi être utilisée pour évaluer le degré de torréfaction du soja.

On prélève des graines lors de la torréfaction et on les broie finement en farine. Les farines (500 mg) sont alors délipidées par addition de 10 ml d'éther de pétrole. Après mélange au vortex, la phase organique est jetée et l'opération est renouvelée une fois. La farine est séchée dans un évaporateur sous vide.

La solubilisation des protéines est réalisée par addition de tampon borate 100 mM à pH 8,0, comme décrit dans l'article de *Bush RF, Touilec R, Caugant I, Guilfoteau P, Effect of raw pea flower on nutrients digestibility and immune responses in the pre-ruminant calf. J. Dairy Sci. 1992, 75 : 3539-3552*.Une centrifugation de 10 mn à 9000 tours par minute permet de séparer les protéines solubies transparentes.

La lecture se fait comme précédemment en diluant 20 fois le surnageant transparent dans de l'eau (soit 150 µl plus 2850 µl d'eau) dans une cuve de fluorescence en acryle.

Dans le tableau II ci-après, on a regroupé des résultats comprenant deux traitements thermiques : farines chauffées au bain-marie et grains chauffés au torréfacteur. Les mesures sur le soja traité au bain-marie sont effectuées sur des échantillons préparés selon la même méthode.

La lecture au spectrofluorimètre est effectuée dans les mêmes conditions qu'à l'exemple 1.

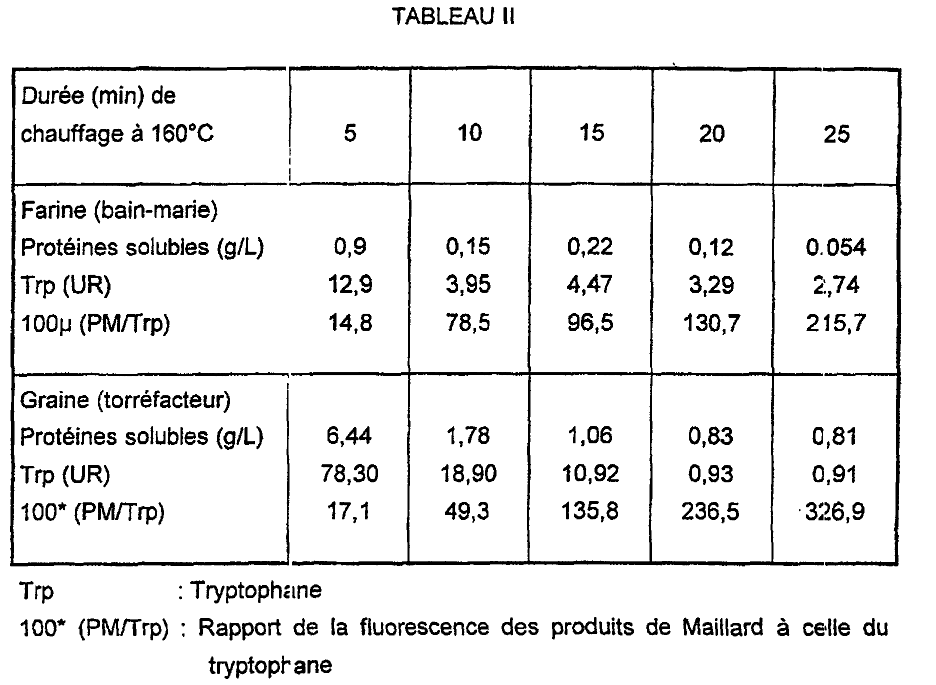

La fluorescence du tryptophane du surnageant transparent est une mesure rapide de la concentration en protéines solubles faiblement dénaturées et est très fortement corrélée au dosage des protéines (Kjeldahl ou méthode colorimétrique du Biuret ; r=0,99). Les produits fluorescents de Maillard sont ici formés à partir des peroxydes lipidiques.

On déduit du présent exemple que la torréfaction de la graine dégrade beaucoup plus fortement les protéines de soja qu'un traitement de la farine au bain-marie, malgré des températures identiques. Le seul dosage des protéines solubles, plus long et coûteux ne permettait pas de tirer ces conclusions.

Cet exemple met donc en évidence la nécessité de conduire la torréfaction dans les meilleures conditions possibles, d'où l'importance de disposer d'une méthode de contrôle aussi rapide et précise que possible comme celle selon la présente invention.

### Exemple 3 : Maïs

La présente méthode peut également servir pour évaluer l'intensité de séchage des grains de maïs, le séchage étant une forme particulière de traitement thermique. Cette intensité de séchage influe sur la qualité et le rendement d'extraction de l'amidon.

Actuellement on mesure cette intensité de séchage par la turbidité de la solution de protéines dénaturées.

Selon la présente invention, on prélève des grains de maïs séchés, on les broie en farine et on solubilise celle-ci dans une solution de chlorure de sodium (NaCI) à 5 %. Puis on centrifuge selon le protocole défini par l'Institut Technique des Céréales et des Fourrages (I.T.C.F.).

Les résultats obtenus sont regroupés dans le tableau III en comparaison avec la méthode traditionnelle exposée ci-dessus.

**TABLEAU III**

| Température de séchage | ambiante | 90°C | 110-120°C |
|---|---|---|---|
| turbidité | 4 % | 47 % | 66 % |
| Trp (UR) | 6,04 | 3,91 | 2,90 |

La faible teneur de sucres réducteurs ou liquides insaturés dans la graine de maïs ne permet pas de mesurer un signal de fluorescence des produits de Maillard. Dans ce cas, seule la première mesure est nécessaire. Elle renseigne sur la dénaturation des protéines à la chaleur en dosant la concentration en protéines encore solubles dans le tampon NaCI.

La méthode selon la présente invention permet donc, d'une part, de contrôler le traitement thermique auquel est soumis un lait ou un aliment protéique et même de le déterminer après coup et, d'autre part, de définir la qualité nutritionnelle de l'aliment analysé.

## Revendications

1. Méthode pour évaluer le traitement thermique auquel est soumis un aliment protéique tel qu'un lait, **caractérisée par le fait qu'**elle comprend les étapes suivantes :
a) on prélève un échantillon dudit aliment auquel on ajoute un tampon de force ionique et de pH appropriés pour obtenir, d'une part, un précipité de protéines dénaturées par ledit traitement thermique et, d'autre part, un surnageant transparent qui renferme des protéines encore solubles ;
b) on dose le tryptophane présent dans ledit surnageant ; et
c) on dose les sous-produits peptidiques fluorescents issus de la réaction avancée de Maillard présents dans ledit surnageant.

2. Méthode selon la revendication 1, **caractérisée par le fait que** le dosage du tryptophane est effectué par sa fluorescence.

3. Méthode selon la revendication 2, **caractérisée par le fait que** l'on effectue le dosage à une longueur d'onde d'excitation de 290 nm et à une longueur d'onde d'émission comprise entre 330 et 350 nm.

4. Méthode selon les revendications 1 à 3, **caractérisée par le fait que** le dosage des sous-produits peptidiques fluorescents issus de la réaction avancée de Maillard présents dans le surnageant, est réalisé avec une longueur d'onde d'excitation de 350 nm et d'emission comprise entre 420 et 440 nm.

## Claims

1. A method for evaluating the heat treatment to which a proteinic nutrient such as milk is subjected, **characterised in that** it comprises the following steps :
a) taking a sample of said nutrient and adding thereto an ionic strength buffer and at appropriate pH to obtain, on the one hand, a precipitate of proteins denatured by said heat treatment and, on the other hand, a transparent supernatant containing proteins which are still soluble ;
b) analysing the tryptophan present in the said supernatant ; and
c) analysing the fluorescent peptide by-products derived from the advanced Maillard reaction present in the said supernatant.

2. A method according to Claim 1, **characterised in that** the analysis of tryptophan is carried out by its fluorescence.

3. A method according to Claim 2, **characterised in that** the analysis is carried out at an excitation wavelength of 290 nm and an emission wavelength of between 330 and 350 nm.

4. A method according to Claims 1 to 3, characterrised in that the analysis of the fluorescent peptide by-products derived from the advanced Maillard reaction pressent in the supernatant is carried out at an excitation wavelength of 350 nm and an emission wavelength of between 420 and 440 nm.

## Patentansprüche

1. Verfahren zur Bewertung der thermischen Behandlung, der ein proteinhaltiges Lebensmittel, wie Milch, unterzogen worden ist, **dadurch gekennzeichnet, dass** es die folgenden Stufen umfasst :
a) man nimmt eine Probe des Lebensmittels und versetzt die Probe mit einem Puffer, dessen Ionenstärke und pH-Wert geeignet sind, um einerseits einen Niederschlag von durch die thermische Behandlung denaturierten Proteinen und andererseits einen durchsichtigen Überstand, in dem die noch löslichen Proteine enthalten sind, zu erhalten ;
b) man bestimmt das im Überstand vorhandene Tryptophan ; und
c) man bestimmt die im Überstand vorhandenen fluoreszierenden Peptidnebenprodukte, die durch die Maillard-Reaktion im fortgeschrittenen Stadium entstanden sind,

2. Verfahren nach Anspruch 1, **dadurch gekennzeichnet, dass** die Bestimmung des Tryptophans aufgrund seiner Fluoreszenz durchgeführt wird.

3. Verfahren nach Anspruch 2, **dadurch gekennzeichnet, dass** die Bestimmung bei einer Anregungswellenlänge von 290 nm und einer Emissionswellenlänge von 330 bis 350 nm durchgeführt wird.

4. Verfahren nach den Ansprüchen 1 bis 3, **dadurch gekennzeichnet, dass** die Bestimmung der im Überstand vorhandenen fluoreszierenden Peptidnebenprodukte, die durch die Maillard-Reaktion im fortgeschrittenen Stadium entstanden sind, bei einer Anregungswellenlänge von 350 nm und einer Emissionswellenlänge von 420 bis 440 nm durchgeführt wird.
